(19) Europäisches Patentamt
European Patent Office
Office européen des brevets

(11) Veröffentlichungsnummer: **0 018 583**
**B1**

(12) # EUROPÄISCHE PATENTSCHRIFT

(45) Veröffentlichungstag der Patentschrift: **15.02.84**

(51) Int. Cl.³: **C 07 C 125/06**

(21) Anmeldenummer: **80102179.1**

(22) Anmeldetag: **23.04.80**

(54) **Verfahren zur Herstellung von Aryl-mono-,-di- und/oder-polyurethanen.**

(30) Priorität: **30.04.79 DE 2917568**
**20.10.79 DE 2942510**

(43) Veröffentlichungstag der Anmeldung:
**12.11.80 Patentblatt 80/23**

(45) Bekanntmachung des Hinweises auf die Patenterteilung:
**15.02.84 Patentblatt 84/7**

(84) Benannte Vertragsstaaten:
**AT BE CH DE FR GB IT LI NL SE**

(56) Entgegenhaltungen:
**Keine**

(73) Patentinhaber: **BASF Aktiengesellschaft,**
**Carl-Bosch-Strasse 38, D-6700 Ludwigshafen (DE)**

(72) Erfinder: **Merger, Franz, Dr., Max-Slevogt-Strasse 25,**
**D-6710 Frankenthal (DE)**
Erfinder: **Towae, Friedrich, Dr., Parkstrasse 22,**
***D-6700 Ludwigshafen* (DE)**

**EP 0 018 583 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Verfahren zur Herstellung von Aryl-mono-, -di- und/oder -polyurethanen

Die Erfindung betrifft ein Verfahren zur Herstellung von Aryl-mono-, -di- und/oder -polyurethanen durch Umsetzung von Mono-, Di- und/oder Polyaminen mit O-Alkylcarbamidsäureestern in Gegenwart mindestens einer Verbindung, die ein oder mehrere Kationen von Metallen aus den Gruppen IA, I, IIA, IIB, IIIA, IIIB, IVA, IVB, VA, VB, VIB, VIIB, VIIIB des Periodensystems besitzt, als Katalysator und gegebenenfalls Harnstoff und Alkoholen bei erhöhten Temperaturen.

N-Arylurethane werden technisch üblicherweise durch Umsetzung von Alkoholen mit aromatischen Isocyanaten oder von aromatischen Aminen mit Chlorkohlensäureestern hergestellt, wobei sowohl die Isocyanate als auch die Chlorkohlensäureester durch Phosgenierung der entsprechenden Amine und Abspaltung von Chlorwasserstoff oder durch Phosgenierung der Alkohole gewonnen werden. (Methoden der organischen Chemie, Houben-Weyl, Band 8, Seiten 137, 120 und 101, Georg-Thieme-Verlag, Stuttgart, 1952).

Diese Verfahren sind technisch sehr aufwendig; ferner bringt die Verwendung von Phosgen wegen damit verbundener Sicherheits- und Umweltschutzmassnahmen erhebliche Nachteile mit sich.

N-Arylurethane finden als Zwischen- und Endprodukte Verwendung. Gemäss DE-OS 2 635 490 oder US-PS 3 919 278 sind sie beispielsweise zur Herstellung von Isocyanaten geeignet. Andere Herstellungsverfahren für N-substituierte Urethane gewinnen daher zunehmend an Bedeutung.

So beschreibt die DE-OS 2 160 111 ein Verfahren zur Herstellung von N-substituierten Urethanen durch Umsetzung eines organischen Carbonats mit einem primären oder sekundären aromatischen Amin in Gegenwart einer Lewissäure. Nachteilig an diesem Verfahren ist, dass organische Carbonate aus Phosgen oder Kohlenmonoxid und Alkohol hergestellt werden und daher teuer sind, die Reaktionsgeschwindigkeit recht gering und die Reaktionszeiten dementsprechend gross sind; ausserdem werden als Nebenprodukte zusätzlich stets N-Alkyl-arylamine erhalten.

Nach Angaben der US-PS 2 834 799 werden Carbamidsäure- und Kohlensäureester durch Umsetzung von Harnstoffen mit Alkoholen in Gegenwart von Bortrifluorid hergestellt. Nachteilig ist hierbei, dass das Bortrifluorid als Katalysator in äquimolaren Mengen benötigt wird, so dass pro erzeugtes Molekül Carbamidsäureester mindestens ein Molekül Bortrifluorid verbraucht wird. Dadurch gestaltet sich das Verfahren nicht nur teuer, sondern es stellt auch eine erhebliche Umweltbelastung dar, da das Bortrifluorid in Form des $H_3N{\cdot}BF_3$-Adduktes anfällt.

Methyl-N-phenylurethan kann ferner aus Kohlenmonoxid, Schwefel, Anilin und Methanol hergestellt werden (R.A. Franz et al., J. Org. Chem. 28, 585 (1963)). Nachteilig sind hierbei insbesondere die geringen Ausbeuten, die auch bei langen Reaktionszeiten nicht über 25% liegen.

Nach Angaben der US-PS 2 409 712 können N-Alkyl- und N-Arylmonourethane durch Umsetzung von Monoaminen mit Harnstoff, N,N'-Dialkyl- oder N,N'-Diarylharnstoff und Alkoholen bei Temperaturen von 150 bis 350 °C, gegebenenfalls unter erhöhtem Druck, hergestellt werden. Beispielhaft beschrieben wird jedoch lediglich die Herstellung von N-Alkylmonourethanen nach der genannten Methode, während N-Phenylurethan durch Alkoholyse von Diphenylharnstoff hergestellt wird.

Zur Herstellung von N-substituierten Monourethanen wird der Harnstoff gemäss US-PS 2 677 698 zunächst mit Monoaminen in den entsprechenden N, N'-disubstituierten Harnstoff übergeführt, dieser wird gereinigt und anschliessend mit Alkohol zur Reaktion gebracht.

Nachteilig an den genannten Verfahren sind neben einer aufwendigen Technik insbesondere die geringen Ausbeuten, die auch durch die Herstellung und Reinigung von N,N'-di-substituierten Harnstoffen nicht verbessert werden können.

Diese Nachteile können auch mit Hilfe des Verfahrens nach US-PS 2 806 051 nicht beseitigt werden. Nach Angaben dieser Patentschrift wird beispielsweise Anilin mit Harnstoff und Alkohol im Molverhältnis 1,0:1,2:2,0 bei Temperaturen unter 200 °C, vorzugsweise von 120 bis 160 °C, umgesetzt. Auch in den vorzugsweise genannten Temperaturbereichen werden in technisch zweckmässigen Reaktionszeiten nach dieser Verfahrensweise N-Phenylmonourethane nur in geringen Ausbeuten erhalten. Es ist daher nicht überraschend, dass in der nachgängigen US-PS 3076007 zur Herstellung von N-Alkyl- und N-Cycloalkylurethanen die obengenannten Methoden unter Verwendung von gegebenenfalls substituierten Harnstoffen nicht beschrieben werden; erwähnt wird hingegen die Umsetzung von Phosgen mit Alkoholen zu Chloralkylformiaten und die anschliessende Weiterreaktion mit Aminen zu Urethanen, während als besonders vorteilhaft zur Herstellung der Urethane die Reaktion von Aminen mit Äthylencarbonat empfohlen wird. Nach einer neueren Verfahrensvariante (DE-OS 2 716 540) werden zur Herstellung von aromatischen Urethanen Dialkylcarbonate mit N-Acylaminen zur Reaktion gebracht.

Es ist ferner bekannt, dass Carbamidsäureäthylester in siedendem Dioxan mit Aminen nicht reagieren (D.G. Crosby and C. Niemann, J. Am. Chem. Soc. 76, 4458 (1954)), und dass N-Alkylurethane mit alkoholischer Ammoniaklösung bei Temperaturen von 160 bis 180 °C eine alkalische Lösung ergeben, aus der nach Neutralisation mit Salzsäure Aminhydrochlorid, Harnstoff, Alkylharnstoff und Alkylurethan isoliert werden kann (M. Brander, Rec. trav. Chim. 37, 88–91 (1917)).

Die genannten Publikationen enthalten keine Angaben über die Reaktion von aromatischen primären Aminen mit Carbamidsäureestern, obwohl aus Ann. 147 (1868) 163 bekannt war, dass durch gemeinsames Erhitzen von Carbamidsäureäthylester mit Anilin bei 160 °C im Bombenrohr Diphenylharnstoff gebildet wird.

Nach Angaben der US-PS 2 409 712 erhält man aus aliphatischen Monoaminen, Harnstoff und Alkohol Alkylurethane nur in geringen Ausbeuten trotz des Einsatzes eines Harnstoffüberschusses. Da gemäss US-PS 2 806 051 bei niedrigerer Temperatur mit weniger Harnstoff etwas höhere Ausbeuten erzielt werden, musste der Fachmann annehmen, dass höhere Molverhältnisse von Harnstoff zu Amin nachteilig sind. Als Nebenprodukte der Synthese von Phenylurethan wurden Diphenylharnstoff und O-Alkylcarbamidsäureester festgestellt. Der O-Alkylcarbamidsäureester wurde hierbei neben nichtumgesetztem Anilin durch Destillation isoliert. Die Bildung von O-Alkylcarbamidsäureester aus Harnstoff und Alkohol wurde demnach als störende Nebenreaktion angesehen.

Da bereits die Herstellung von N-monoalkylsubstituierten Urethanen aus Alkylaminen, Harnstoff und Alkoholen nur in mässigen Ausbeuten gelingt und Carbamidsäureester als Nebenprodukte gebildet werden, ist nicht überraschend, dass durch keine der genannten Publikationen – weder einzeln noch im Kombination – eine Lehre vermittelt wird, die die Herstellung von Aryl-mono-, -di- und/oder -polyurethanen aus Arylaminen und Carbamidsäureestern in Betracht zieht.

Zur Beseitigung dieser Nachteile wurde vorgeschlagen, N-Arylurethane aus Nitroaromaten, Kohlenmonoxid und Alkoholen in Gegenwart von Katalysatoren herzustellen:

Nach Angaben der DE-OS 1 568 044 (US-PS 3 467 694) können Urethane durch Umsetzung von organischen Nitroverbindungen, Kohlenmonoxid und hydroxylhaltigen Verbindungen in Gegenwart eines Katalysators, der aus einem Edelmetall und einer Lewissäure besteht, unter praktisch wasserfreien Bedingungen in Abwesenheit von Wasserstoff bei erhöhtem Druck und Temperaturen über 150 °C hergestellt werden. Gemäss DE-OS 2 343 826 (US-PS 3 895 054) werden Urethane aus hydroxylgruppenhaltigen Verbindungen, Kohlenmonoxid und Nitro-, Nitroso-, Azo- und Azoxygruppen aufweisenden Verbindungen in Gegenwart von Schwefel, Selen, einer Schwefel- und/oder Selenverbindung und mindestens einer Base und/oder Wasser hergestellt. Die DE-OS 2 623 694 (US-PS 4 080 365) beschreibt die Herstellung aromatischer Urethane aus den oben genannten Ausgangsverbindungen in Gegenwart von Selen enthaltenden Katalysatorsystemen sowie speziellen aromatischen Amino- und Harnstoffverbindungen.

Aber auch diese Verfahren weisen noch schwerwiegende Nachteile auf. Das toxische Kohlenmonoxid und Katalysatoren, die toxisch sind bzw. im Laufe der Umsetzung toxische Verbindungen bilden, beispielsweise Selen- und Schwefelwasserstoff, oder Katalysatoren, die sehr teuer und schwierig rückführbar sind, wie Palladium, erfordern einen hohen technischen Aufwand und kostspielige Sicherheitsmassnahmen.

Die Aufgabe der vorliegenden Erfindung bestand darin, Aryl-mono-, -di- und/oder -polyurethane aus leicht zugänglichen Ausgangskomponenten unter wirtschaftlich vertretbaren Bedingungen in guten Raum-Zeit-Ausbeuten und mit hoher Selektivität herzustellen.

Diese Aufgabe wurde gelöst durch ein Verfahren zur Herstellung von Aryl-mono-, -di- und/oder -polyurethanen, das dadurch gekennzeichnet ist, dass man primäre aromatische Mono-, Di- und/oder Polyamine mit O-Alkylcarbamidsäureestern in Gegenwart mindestens einer Verbindung, die ein oder mehrere Kationen von Metallen aus den Gruppen IA, IB, IIA, IIB, IIIA, IIIB, IVA, IVB, VA, VB, VIB, VIIB, VIIIB des Periodensystems besitzt, als Katalysator und gegebenenfalls Harnstoff und Alkoholen bei erhöhten Temperaturen umsetzt und das entstehende Ammoniak gegebenenfalls abtrennt.

Die Reaktion kann durch Gleichung (I) veranschaulicht werden:

$$Art(-NH_2)_n + nH_2N{-}COOR \rightarrow Ar(NHCOOR)_n + nNH_3 \quad (I)$$

Vorzugsweise wird die Umsetzung jedoch in Gegenwart von Harnstoff und Alkohol nach Gleichung (II) durchgeführt:

$$Ar(-NH_2)_n + aH_2N{-}CO{-}NH_2 + b\ ROOC{-}NH_2 + a\ ROH \downarrow Ar(-NHCOOR)_n + (2a+b)\ NH_3 \quad (II)$$

In den Gleichungen (I) und (II) bedeuten n, a und b ganze Zahlen, wobei n für 1 bis 7, vorzugsweise 1 bis 5 steht und nach Gleichung (II) a + b = n und a : n = 1,5–0 sind.

Die Bildung von Aryl-mono-, -di- und/oder -polyurethanen, insbesondere in einem Verfahrensschritt und in guten Ausbeuten, war besonders überraschend, da nach bekannter Lehrmeinung aus Carbamidsäureestern und aromatischen Aminen N,N'-Diarylharnstoffe erhalten werden, die meist aus Alkohol umkristallisiert werden. Unsubstituierte Urethane reagieren in Gegenwart von aromatischen Aminen auch in Alkohol sehr leicht zu N,N'-Diarylharnstoffen weiter (Methoden der organischen Chemie, Houben-Weyl, Band 8, Seiten, 152, 140 und 161, Georg Thieme Verlag, Stuttgar, 1952).

Entsprechend werden aus Diaminen und Harnstoff bei Temperaturen über 100 °C hochmolekulare, verspinnbare Kondensationsprodukte erhalten (DE-PS 896 412). Hochmolekulare Polyharnstoffe, beispielsweise mit Molekulargewichten von 8000 bis 10 000 und grösser, werden auch erhalten, wenn man Diurethane mit Diaminen bei Temperaturen von ungefähr 150 bis 300 °C kondensiert (US-PS 2 181 663 und US-PS 2 568 885). Da Mono- und Polyurethane ausserdem thermisch in Isocyanate, Alkohole und gegebenenfalls Olefine, Kohlendioxid, Harnstoff und Carbodiimide gespalten werden, wobei die erhaltenen Spaltprodukte wieder zahlreiche Folgeprodukte, z.B. Biurete, Allophanate, Isocyanurate, Polycarbodiimide u.a. bilden können (J.A.C.S. 80, 5495 (1958) und J.A.C.S. 78, 1946 (1956)), konnte nicht erwartet werden,

dass unter sehr ähnlichen Reaktionsbedingungen nach dem erfindungsgemässen Verfahren Aryl-mono-, -di- und/oder -polyurethane in sehr guten Ausbeuten erhalten werden.

Es ist insbesondere überraschend, dass mit den genannten Katalysatoren Aryl-mono-, -di- und/oder -polyurethane in sehr guten Ausbeuten bereits aus aromatischen primären Aminen und O-Alkylcarbamidsäureestern ohne Zusatz von Alkohol erhalten werden, überschüssige O-Alkylcarbamidsäureester nicht zu wesentlichen Zersetzungen und Polykondensationen führen, die erfindungsgemässe Reaktion bereits bei tieferen Temperaturen technisch durchgeführt und auch in ihrer Selektivität noch verbessert werden kann. Überraschend ist ferner, dass die Umsetzung mit Harnstoff und Alkohol in Gegenwart mindestens eines der genannten Katalysatoren bereits bei tieferen Temperaturen technisch erheblich effektiver wird, wenn sie zusammen mit grösseren Mengen O-Alkylcarbamidsäureester durchgeführt wird. Hierbei ist nicht erforderlich, O-Alkylcarbamidsäureester separat herzustellen. In einer leicht praktikablen, vorzugsweise zur Anwendung kommenden Ausführungsform wird O-Alkylcarbamidsäureester zusammen mit Harnstoff und Alkohol eingesetzt und nach weitgehender bis vollständiger Umsetzung des aromatischen Mono-, Di- und/oder Polyamins durch Destillation abgetrennt und gegebenenfalls zurückgeführt. Das erfindungsgemässe verfahren kann auch kontinuierlich durchgeführt werden.

Für die erfindungsgemässe Umsetzung mit O-Alkylcarbamidsäureestern in Gegenwart von Katalysatoren und gegebenenfalls Harnstoff und Alkohol eignen sich gegebenenfalls substituierte, primäre aromatische Mono-, Di- und Polyamine. Im einzelnen seien beispielhaft genannt:

Aromatische Monoamine, wie Anilin, substituierte Aniline, wie in 2-, 3- und/oder 4-Stellung durch eine Nitro-, Methyl-, n-Propyl-, Isopropyl-, n-Butyl-, Isobutyl-, sek.-Butyl-, tert.-Butylgruppe oder ein Chloratom substituierte Aniline; ortho-, meta- und/oder para-Hydroxi-, Methoxi-, Äthoxi-, Propoxi-, Isopropoxi-, N-Butoxi-, Isobutoxi-, sek.-Butoxi- und tert.-Butoxianilin; durch eine Aminogruppe in m- und/oder p-Stellung substituierte Benzoesäurealkylester mit 1 bis 4 Kohlenstoffatomen im Alkylrest, durch eine Aminogruppe in m- und/oder p-Stellung substituierte N-Alkoxycarbonylaminobenzole und -toluole mit 1 bis 4 Kohlenstoffatomen im Alkylrest; α- und β-Naphthylamin; aromatische Diamine, wie 1,3- und 1,4-Diaminobenzol; durch eine Nitro-, Methyl-, Äthyl-, n-Propyl-, Isopropyl-, n-Butyl-, Isobutyl-, sek.- Butyl-, tert.-Butyl-, Methoxy-, Äthoxy-, n-Propoxy-, Isopropoxy-, n-Butoxy-, Isobutoxy-, sek.-Butoxy-, tert.-Butoxygruppe oder ein Halogenatom, vorzugsweise ein Fluor- und/oder Chloratom, in 2- und/oder 4-Stellung substituiertes 1,3-Diaminobenzol oder in 2-Stellung substituiertes 1,4-Diaminobenzol, 1,5- und 1,8-Diaminonaphthalin, 4,4'-Diaminodiphenyl, 2,2'-, 2,4'- und 4,4'-Diamino-diphenylmethan, und die entsprechenden Isomerengemische und aromatische Polyamine, wie 1,3,5-Triaminobenzol, 2,4,6-Triaminotoluol und 1,3,5-Triaminonaphthalin, Polyphenyl-polymethylen-polyamine sowie Mischungen aus Diamino-diphenylmethanen und Polyphenyl-polymethylen-polyaminen, die nach bekannten Verfahren durch Kondensation von Anilin und Formaldehyd in Gegenwart von vorzugsweise Mineralsäuren als Katalysatoren erhalten werden.

Besonders bewährt haben sich und daher vorzugsweise verwendet werden als aromatische Monoamine o-, m- und/oder p-Toluidin, o-, m- und/oder p-Anisidin, 3-Hydroxianilin, o-, m- und/oder p-Chloranilin, 2,4-, 3,4- und 3,5-Dichloranilin, 2-Nitro-4-aminotoluol, 4-Nitro-2-aminotoluol, 2-Nitro-6-amino-toluol und N-Alkoxicarbonyl-arylamine der Formel

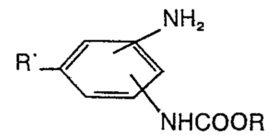

in der R einen Methyl-, Äthyl-, Propyl-, Isopropyl-, n-Butyl-, Isobutyl-, sek.-Butyl- oder tert.-Butylrest und R' ein Wasserstoffatom oder den Rest R bedeuten, sowie insbesondere Anilin, als aromatische Diamine, 3,3'-Ditoluylen-4,4'-diamin, 2,4- und 2,6-Toluylendiamin, sowie die entsprechenden Isomerengemische, 2,2'-, 2,4'- und 4,4'-Diaminodiphenylmethane und die entsprechenden Isomerengemische, 1,5- und 1,8-Naphthylen-diamin und als Polyamine Mischungen aus Diamino-diphenyl-methanen und Polyphenyl-polymethylen-polyaminen.

Bei der Reaktion werden die Aminogruppen in Alkoxicarbonylaminogruppen übergeführt, unabhängig davon, ob die übrigen Substituenten unverändert und erhalten bleiben oder ebenfalls umgewandelt werden.

Geeignete O-Alkylcarbamidsäureester besitzen die Formel $H_2N{-}COOR$, in der R einen gegebenenfalls substituierten aliphatischen, cycloaliphatischen oder aromatisch-aliphatischen Rest bedeutet. In Betracht kommen beispielsweise O-Alkylcarbamidsäureester auf Basis von primären aliphatischen Monoalkoholen mit 1 bis 20 Kohlenstoffatomen, vorzugsweise 1 bis 10 Kohlenstoffatomen, wie Carbamidsäure-methylester, -äthylester, -propylester, -n-butylester, -isobutylester, -2- und -3-methylbutylester, -neopentylester, -pentylester, -2-methyl-pentylester, n-hexylester -2-äthylhexylester, -heptylester, -n-octylester, -n-nonylester, -n-decylester und -n-dodecylester, -2-phenylpropylester und -benzylester auf Basis von sekundären aliphatischen und cycloaliphatischen Monoalkoholen mit 3 bis 15 Kohlenstoffatomen, vorzugsweise 3 bis 6 Kohlenstoffatomen, wie Carbamidsäure-isopropylester, -sek.-butylester, -sek.-iso-amylester, -cyclopentylester, -cyclohexyl-ester, tert.-butyl-cyclohexylester und -bicyclo-(2,2,1)-heptylester. Vorzugsweise verwendet werden Carbamidsäure-methylester, -äthylester, -propylester, -butylester, -isobutylester, -2- und -3-methylbutylester, -pentylester, -hexylester, -2-äthylhexylester, -heptylester, -octylester und -cyclohexylester.

Zur Herstellung der Aryl-mono-, -di- und/oder -polyurethane nach dem erfindungsgemässen Verfahren werden die obengenannten primären aromatischen Mono-, Di- und/oder Polyamine mit den O-Alkylcarbamidsäureestern und gegebenenfalls Alkohlen in solchen Mengen zur Reaktion gebracht, dass das Verhältnis von $NH_2$-Gruppen der primären aromatischen Amine zu O-Alkylcarbamidsäureester zu Alkohol 1:0,5–20:0–100, vorzugsweise 1:0,8–10:0–30 und insbesondere für Arylmonourethane 1.1–6:0–5 und für Aryl-di- und/oder -polyurethane 1:1–6:2–20 beträgt.

Wie bereits ausgeführt wurde, werden zur Herstellung der Aryl-mono-, die- und/oder -polyurethane in einer der bevorzugten Verfahrensvarianten neben O-Alkylcarbamidsäureestern zusätzlich Harnstoff und Alkohol mitverwendet, wobei das Verhältnis von $NH_2$-Gruppen der primären aromatischen Amine zur Summe aus O-Alkyl carbamidsäureester und Harnstoff ebenfalls 1:0,5–20, vorzugsweise 1:0,8–10 und insbesondere 1:1–6 beträgt, das Molverhältnis von Harnstoff zu Aminogruppen der primären aromatischen Amine gleich oder kleiner als 1,5, vorzugsweise 1,25–0,75 und das Molverhältnis Harnstoff zu Alkohol gleich oder kleiner als 1 sind.

Harnstoff wird zweckmässigerweise in handelsüblicher Form und Reinheit eingesetzt.

Als Alkohole können beliebige, gegebenenfalls substituierte primäre oder sekundäre aliphatische Alkohole sowie Mischungen davon verwendet werden. Vorzugsweise wird der dem O-Alkylcarbamidsäureester entsprechende Alkohol eingesetzt.

In Betracht kommen beispielsweise primäre aliphatische Alkohole mit 1 bis 20 Kohlenstoffatomen, vorzugsweise 1 bis 10 Kohlenstoffatomen, wie Methanol, Äthanol, Propanol, n-Butanol, 2-Methylbutanol, n-Pentanol, Neopentylalkohol, 2-Methyl-pentanol, n-Hexanol, n-Heptanol, n-Octanol, Nonanol, n-Decanol und n-Dodecanol, sekundäre aliphatische und cycloaliphatische Alkohole mit 3 bis 15 Kohlenstoffatomen, vorzugsweise 3 bis 6 Kohlenstoffatomen, wie Isopropanol, sec.-Butanol, sec.-Isoamylalkohol, Cyclopentanol, 2,3- oder 4-Methyl-cyclohexanol, Cyclohexanol und Bicyclo-[2,2,1]-heptanol. Vorzugsweise verwendet werden als Monoalkohole Methanol, Äthanol, Propanol, n-Butanol, Isobutanol, 2-Äthyl-butanol, 2- und 3-Methylbutanol, n-Pentanol, n-Hexanol, 2-Äthyl-hexanol, Heptanol, Octanol und Cyclohexanol.

Die Alkohole können gegebenenfalls mit anderen unter den Reaktionsbedingungen inerten organischen Lösungsmitteln gemischt werden.

Erfindungsgemäss wird die Reaktion in Gegenwart eines oder mehrerer Katalysatoren durchgeführt. Als Katalysatoren eignen sich anorganische und organische Verbindungen, die ein oder mehrere, vorzugsweise ein Kation von Metallen der Gruppen IA, IB, IIA, IIB, IIIA, IIIB, IVA, IVB, VA, VB, VIB, VIIB und VIIIB des Periodensystems, definiert gemäss Handbook of Chemistry and Physics 14th Edition, publiziert von Chemical Rubber Publishing Co. 2310 Superior Ave. N.E. Cleveland, Ohio, enthalten, beispielsweise Halogenide, wie Chloride und Bromide, Sulfate, Phosphate, Nitrate, Borate, Alkoholate, Phenolate, Sulfonate, Oxide, Oxidhydrate, Hydroxide, Carboxylate, Chelate, Carbonate und Thio- oder Dithiocarbamate. Beispielhaft genannt seien die Kationen folgender Metalle: Lithium, Natrium, Kalium, Magnesium, Calcium, Aluminium, Gallium, Zinn, Blei, Wismut, Antimon, Kupfer, Silber, Gold, Zink, Quecksilber, Cer, Titan, Vanadium, Chrom, Molybdän, Mangan, Eisen, Kobalt und Nickel. Vorzugsweise Verwendung finden die Kationen von Lithium, Calcium, Aluminium, Zinn, Wismut, Antimon, Kupfer, Zink, Titan, Vanadium, Chrom, Molybdän, Mangan, Eisen und Kobalt. Die Katalysatoren können ohne erkennbare deutliche Nachteile auch in Form ihrer Hydrate oder Ammoniakate zum Einsatz kommen.

Als typische Katalysatoren seien beispielhaft folgende Verbindungen genannt: Lithiummethanolat, Lithiummäthanolat, Lithiumpropanolat, Lithiumbutanolat, Natriummethanolat, Kalium-tert.-butanolat, Magnesiummethanolat, Calciummethanolat, Zinn-(II)-chlorid, Zinn-(IV)-chlorid, Bleiacetat, Bleiphosphat, Antimon-(III)-chlorid, Antimon-(V)-chlorid, Aluminium-isobutylat, Aluminiumtrichlorid, Wismut-(III)-chlorid, Kupfer-(II)-acetat, Kupfer-(II)-sulfat, Kupfer-(II)-nitrat, Bis-(triphenylphosphinoxido)-kupfer-(II)-chlorid, Kupfermolybdat, Silberacetat, Goldacetat, Zinkoxid, Zinkchlorid, Zinkacetat, Zinkacetonylacetat, Zinkoctoat, Zinkoxalat, Zinkhexylat, Zinkbenzoat, Zinkundecylenat, Cer-(IV)-oxid, Uranylacetat, Titan-tetrabutanolat, Titantetrachlorid, Titantetraphenolat, Titannaphthenat, Vanadium-(III)-chlorid, Vanadiumacetonylacetat, Chrom-(III)-chlorid, Molybdän-(VI)-oxid, Molybdänacetylacetonat, Wolfram-(VI)-oxid, Mangan-(II)-chlorid, Mangan-(II)-acetat, Mangan-(III)-acetat, Eisen-(II)-acetat, Eisen(III)-acetat, Eisenphosphat, Eisenoxalat, Eisen-(III)-chlorid, Eisen-(III)-bromid, Kobaltacetat, Kobaltchlorid, Kobaltsulfat, Kobaltnaphthenat, Nickelchlorid, Nickelacetat und Nickelnaphthenat sowie deren Gemische.

Die Katalysatoren werden zweckmässigerweise in Mengen, die 0,001 bis 0,1, vorzugsweise 0,0005 bis 0,05 Äquivalenten des Metallkations, bezogen auf die $NH_2$-Gruppen der aromatischen Mono-, Di- und Polyamine entsprechen, verwendet. Die Metallionen können auch gebunden an Ionenaustauscher in heterogener Phase eingesetzt werden.

Die Umsetzung wird bei erhöhten Temperaturen, beispielsweise bei Temperaturen von 100 bis 250 °C, vorzugsweise 120 bis 210 °C und insbesondere 135 bis 190 °C und Drücken von 0,1 bis 120 bar, vorzugsweise 0,5 bis 60 bar, insbesondere 1 bis 40 bar, durchgeführt, wobei es sich als vorteilhaft erwiesen hat, das entstehende Ammoniak aus der Reaktionsmischung, beispielsweise durch Destillation, abzutrennen. Bei gegebener Temperatur wird die Umsetzung dann vorzugsweise unter einem Druck durchgeführt, bei dem das entstehende Ammoniak selektiv aus dem Reaktionsgemisch abdestilliert werden kann. Die entsprechenden Werte können Tabellen mit physikalischen Kenndaten des Ammoniaks und der Alkohole entnommen werden. Für den genannten Temperaturbereich ergeben sich Reaktionszeiten von 0,5 bis 100 Stunden, vorzugsweise von 1 bis 50 Stunden, und insbesondere 2 bis 25 Stunden.

Die Aryl-mono-, -di- und/oder -polyurethane werden nach dem erfindungsgemässen Verfahren zweckmässigerweise wie folgt hergestellt. Die primären aromatischen Mono-, Di- und/oder Polyamine, O-Alkylcarbamidsäureester und gegebenenfalls Alkohole bzw. vorzugsweise Mono-, Di- und/oder Polyamine, O-Alkylcarbamidsäureester, Harnstoff und Alkohole werden in den genannten Mengenverhältnissen gemischt und in Gegenwart von mindestens einem Katalysator in einem Reaktionsgefäss, ausgestattet mit einer Vorrichtung zur Abtrennung des Ammoniaks, gegebenenfalls unter Rühren erhitzt. Das entstehende Ammoniak kann nach beendeter Umsetzung abgetrennt werden; vorzugsweise wird es jedoch bereits im Laufe der Umsetzung kontinuierlich oder absatzweise abdestilliert. Hierbei kann es zweckmässig sein, insbesondere bei der Umsetzung in Gegenwart von niedermolekularen Alkoholen unter Druck, das Ammoniak mit Hilfe eines unter den Reaktionsbedingungen inerten Schleppmittels, beispielsweise eines Gases, wie Stickstoff oder einem Teil des Alkohols abzutrennen. Aus der erhaltenen Reaktionsmischung wird anschliessend, gegebenenfalls nach Abtrennung des Katalysators und Abfiltrieren von Feststoffen das Aryl-mono-, -di- und/oder -polyurethan isoliert, beispielsweise durch Abdestillieren des überschüssigen O-Alkylcarbamidsäureesters und/oder Alkohols, durch partielles Abdestillieren des überschüssigen O-Alkylcarbamidsäureesters und/oder Alkohols und Auskristallisieren, durch Ausfällen mit oder auch durch Umkristallisieren aus anderen Lösungsmitteln. Der abgetrennte O-Alkylcarbamidsäureester kann gegebenenfalls zurückgeführt werden.

Die erhaltenen Aryl-mono-, -di- und/oder -polyurethane sind wertvolle End- und Zwischenprodukte. Als Endprodukte werden sie beispielsweise als Schädlingsbekämpfungsmittel verwendet. Als Zwischenprodukte werden sie beispielsweise als Komponenten für Polykondensations- und Polymersysteme eingesetzt, insbesondere jedoch unter Abspaltung von Alkohol in die entsprechenden Isocyanate übergeführt, wobei Di- und Poly-isocyanate zur Herstellung von Polyurethanen Anwendung finden.

Die in den Beispielen genannten Teile beziehen sich auf das Gewicht. Die Elementarzusammensetzungen und Strukturen sind durch Elementaranalyse, Massenspektroskopie sowie IR- und NMR-Spektren bestätigt.

Beispiel 1

93 Teile Anilin werden mit 257 Teilen Carbamidsäurebutylester, 0,9 Teilen Kobaltacetat und 250 Teilen Butanol 8 Stunden lang auf 175 °C erhitzt, wobei über ein Druckventil im Reaktionsgefäss ein Druck von 4 bis 5 bar eingestellt wird. Das während der Reaktion gebildete Ammoniak wird unter Verwendung von 25 l Stickstoff je l Reaktionsgemisch und Stunde als Schleppmittel kontinuierlich abdestilliert. Nach beendeter Reaktion zieht man nicht umgesetztes Anilin, überschüssiges Butanol und überschüssigen Carbamidsäurebutylester bei ca. 20 mbar ab und erhält durch Destillation bei 145–148 °C, 0,1 mbar, 172 Teile Phenylbutylurethan (98% der Theorie, bezogen auf umgesetztes Anilin) vom Schmp. 80–83 °C. Der Umsatz an Anilin beträgt 91%.

Beispiel 2

93 Teile Anilin werden mit 450 Teilen Carbamidsäuremethylester, 0,9 Teilen Kobaltacetat und 96 Teilen Methanol 6 Stunden lang auf 175 °C erhitzt, wobei über ein Druckventil im Reaktionsgefäss ein Druck von 5 bis 6 bar eingestellt wird. Das während der Reaktion gebildete Ammoniak wird portionsweise abdestilliert. Nach beendeter Reaktion wird die Reaktionsmischung gaschromatographisch nach der Methode des «Internen Standard» analysiert. Es zeigt sich, dass 140 Teile Phenylmethylurethan (98,6% der Theorie, bezogen auf umgesetztes Anilin) gebildet werden. Der Umsatz an Anilin beträgt 94%.

Vergleichsbeispiel

Man verfährt analog den Angaben von Beispiel 2, setzt der Reaktionsmischung jedoch keinen Katalysator zu. Die gaschromatographische Analyse zeigt, dass 113 Teile Phenylmethylurethan (88% der Theorie, bezogen auf umgesetztes Anilin) sowie 7 Teile N-Methylanilin (7,7% der Theorie, bezogen auf umgesetzten Anilin) gebildet werden. Der Umsatz an Anilin beträgt 85%.

Beispiel 3

18 Teile Anilin werden mit 36,5 Teilen Carbamidsäuremethylester und 0,2 Teilen Vanadiumtrichlorid in 75 Teilen Diäthylenglykoldimethyläther 3 Stunden lang zum Sieden erhitzt. Nach beendeter Reaktion wird die Reaktionsmischung gaschromatographisch nach der Methode des «Internen Standard» analysiert. Es zeigt sich, dass 19 Teile Phenylmethylurethan (73,9% der Theorie, bezogen auf umgesetztes Anilin) entstanden sind. Der Umsatz an Anilin beträgt 88%.

Beispiel 4

93 Teile Anilin werden mit 240 Teilen Carbamidsäurebutylester und 0,9 Teilen Vanadiumtrichlorid in 86 Teilen Butanol 16 Stunden lang auf 135–140 °C erhitzt. Nach beendeter Reaktion wird die Reaktionsmischung gaschromatographisch analysiert. Es zeigt sich, dass 176 Teile Phenylbutylurethan (97% der Theorie, bezogen auf umgesetztes Anilin) gebildet werden. Der Umsatz an Anilin beträgt 94%. Durch fraktionierende Destillation werden 164 Teile Phenylbutylurethan bei einem Siedepunkt von 138–144 °C bei 0,03 mbar isoliert.

Vergleichsbeispiel

Man verfährt analog Beispiel 4, jedoch ohne

Katalysatorzusatz. Die gaschromatographische Analyse zeigt, dass 6,7 Teile Phenylbutylurethan entstanden sind (87% der Theorie, bezogen auf umgesetztes Anilin); der Umsatz an Anilin beträgt jedoch nur 4%.

Beispiele 5 bis 35

Man verfährt analog den Angaben von Beispiel 4, verwendet jedoch anstelle von Vanadiumtrichlorid andere Verbindungen mit Metallkationen als Katalysatoren.

Die eingesetzten Katalysatoren sowie die erhaltenen Ausbeuten und Umsätze sind in der folgenden Tabelle zusammengefasst:

In der Tabelle bedeuten:

| | |
|---|---|
| $C_2H_3O_2$ | Acetatrest |
| $[(C_6H_5)_3PO]_2$ | Bis-(triphenylphosphinoxid) |
| $C_2O_4$ | Oxalatrest |
| $C_{11}H_{19}O_2$ | Undecylenatrest |
| $C_5H_7O_2$ | Acetylacetonatrest |
| $D_4H_9O$ | Butylatrest |
| $CH_3O$ | Methylatrest |
| n-$C_3H_7O$ | n-Propylatrest |
| iso-$C_3H_7O$ | iso-Propylatrest |

Tabelle

| Beispiel Nr. | Katalysator | Umsatz an Anilin % | Phenyl-butylurethan Ausbeute: % |
|---|---|---|---|
| 5 | $MnCl_2$ | 31 | 98 |
| 6 | $Mn(C_2H_3O_2)_2 \times H_2O$ | 31 | 58 |
| 7 | $Mn(C_2H_3O_2)_3 \times H_2O$ | 37 | 72 |
| 8 | $CoCl_2$ | 67 | 51 |
| 9 | $Co(C_2H_3O_2)_2 \times 4\ H_2O$ | 89 | 99 |
| 10 | $CoSO_4 \times 7\ H_2O$ | 58 | 85 |
| 11 | $Cu(NO_3)_2 \times 3\ H_3O$ | 33 | 72 |
| 12 | $Cu(C_2H_3O_2)_2 \times H_2O$ | 71 | 86 |
| 13 | $Cu[(C_6H_5)_3PO]_2Cl_2$ | 70 | 84 |
| 14 | Ni-naphthenat | 57 | 87 |
| 15 | $Fe(OH)(C_2H_3O_2)_2$ | 82 | 99 |
| 16 | $Fe(C_2H_3O_2)_2$ | 91 | 99 |
| 17 | $FePO_4$ | 83 | 93 |
| 18 | $Fe(C_2O_4) \times 2\ H_2O$ | 54 | 95 |
| 19 | $Zn(C_2H_3O_2)_2 \times 2\ H_2O$ | 72 | 100 |
| 20 | $ZnCl_2$ | 40 | 90 |
| 21 | $Zn(C_{11}H_{19}O_2)_2$ | 74 | 98 |
| 22 | $MoO_2(C_5H_7O_2)_2$ | 85 | 96 |
| 23 | $MoO_3$ | 64 | 88 |
| 24 | $SbCl_5$ | 87 | 88 |
| 25 | $CrCl_3$ | 69 | 97 |
| 26 | $SnCl_2$ | 90 | 88 |
| 27 | $SnCl_4$ | 92 | 79 |
| 28 | $BiCl_3$ | 47 | 85 |
| 29 | $TiCl_4$ | 50 | 98 |
| 30 | $Ti(C_4H_9O)_4$ | 64 | 94 |
| 31 | $LiC_4H_9O$ | 67 | 83 |
| 32 | $NaCH_3O$ | 16 | 99 |
| 33 | $Ca(n\text{-}C_3H_7O)_2$ | 58 | 97 |
| 34 | $Al(iso\text{-}C_3H_7O)_3$ | 67 | 93 |
| 35 | $CeO_2$ | 12 | 97 |

Beispiel 36

12,2 Teile 2,4-Diaminotoluol werden mit 22,3 Teilen Carbamidsäureäthylester, 1 Teil Eisen-(II)-acetat und 28 Teilen Äthanol 6 Stunden lang auf 180 °C erhitzt, wobei über ein Druckregelventil im Reaktionsgefäss ein Druck von 14–17 bar eingestellt wird. Das während der Reaktion gebildete Ammoniak wird unter Verwendung von 30 l Stickstoff je l Reaktionsgemisch und Stunde als Schleppmittel kontinuierlich abdestilliert. Nach beendeter Reaktion analysiert man die Reaktionsmischung durch Hochdruck-Flüssigchromatographie nach der Methode des «Externen Standard». Es zeigt sich, dass 19 Teile 2,4-Di-(äthoxicarbonylamino)-toluol (73,6% der Theorie, bezogen auf umgesetztes 2,4-Diaminotoluol) und 3,6 Teile eines Gemisches aus 2-Amino-4-(äthoxycarbonylamino)-toluol und 4-Amino-2-(äthoxycarbonylamino)-toluol (19,1% der Theorie, bezogen auf umgesetztes 2,4-Diaminotoluol) entstanden sind. Der Umsatz des 2,4-Diaminotoluol beträgt 97%.

Um das 2,4-Di-(äthoxycarbonylamino)-toluol zu isolieren, destilliert man überschüssiges Äthanol und überschüssigen Carbamidsäureäthylester unter vermindertem Druck bei 10 mbar ab, löst den Rückstand in 250 Teilen Methylenchlorid und

wäscht ihn mehrmals mit Wasser. Danach trennt man das Methylenchlorid ab, gibt 50 Teile Äthanol hinzu und lässt die Mischung in einer Eis-Kochsalz-Mischung abkühlen. Nach einiger Zeit kristallisiert 2,4-Di-(äthoxycarbonylamino)-toluol mit einem Schmelzpunkt von 108-110°C aus.

Beispiel 37

7,9 Teile 1,5-Diaminonaphthalin werden mit 24,5 Teilen Carbamidsäureäthylester, 3 Teilen Harnstoff, 0,15 Teilen Uranylacetat und 34 Teilen Äthanol 12 Stunden lang auf 180 °C erhitzt, wobei über ein Druckventil im Reaktionsgefäss ein Druck von 16–18 bar eingestellt wird. Das während der Reaktion gebildete Ammoniak wird unter Verwendung von 20 l Stickstoff je l Reaktionsgemisch und Stunde als Schleppmittel kontinuierlich abdestilliert. Nach beendeter Reaktion wird die Reaktionsmischung durch Hochdruck-Flüssigchromatographie nach der Methode des «Externen Standard» analysiert. Es zeigt sich, dass 9 Teile 1,5-Bis-(äthoxicarbonylamino)-naphthalin (83,9% der Theorie, bezogen auf umgesetztes 1,5-Diaminonaphthalin) vom Schmp. 221–224 °C entstanden sind. Der Umsatz an 1,5-Diaminonaphthalin beträgt 71%.

Beispiel 38

120 Teile 3,5-Dichloranilin werden mit 220 Teilen Carbamidsäuremethylester und 6 Teilen Kobaltacetat in 100 Teilen Diäthylenglykoldimethyläther 10 Stunden lang unter Rückfluss erhitzt. Nach beendeter Reaktion werden überschüssiger Carbamidsäuremethylester und Diäthylenglykoldimethyläther unter vermindertem Druck bei 15 mbar abdestilliert, der Rückstand in 500 Teilen Methylenchlorid gelöst, mit 60 Teilen 10%iger Schwefelsäure und mit 3mal 100 Teilen Wasser gewaschen. Die organische Phase wird getrocknet und eingeengt, wobei 123 Teile 3,5-Dichlorphenylmethylurethan (90,0% der Theorie, bezogen auf umgesetztes 3,5-Dichloranilin) vom Schmelzpunkt 117–119 °C erhalten werden. Der Umsatz an 3,5-Dichloranilin beträgt 83%.

Beispiel 39

10,0 Teile 4,4′-Diaminodiphenylmethan werden mit 143 Teilen Carbamidsäureoctylester, 6 Teilen Harnstoff und 0,3 Teilen Kobaltacetat in 150 Teilen Octanol 6 Stunden lang zum Sieden erhitzt. Das während der Reaktion gebildete Ammoniak wird unter Verwendung von 10 l Stickstoff je l Reaktionsgemisch und Stunde als Schleppmittel kontinuierlich abdestilliert. Nach dem Erkalten kristallisieren 16 Teile 4,4′-Di(octoxicarbonylamino)-diphenylmethan (71,4% der Theorie, bezogen auf umgesetztes 4,4′-Diaminodiphenylmethan) vom Schmp. 117–119 °C aus. Der Umsatz des 4,4′-Diaminodiphenylmethan beträgt 87%. In der Mutterlauge befindet sich noch 4-Amino-4′-(octoxicarbonylamino)-di-phenylmethan.

Beispiel 40

Man verfährt analog den Angaben von Beispiel 39, ohne zusätzlich Harnstoff mitzuverwenden. Man erhält 11 Teile 4,4′-Di-(octoxicarbonylamino)-diphenylmethan (61,9% der Theorie, bezogen auf umgesetztes 4,4′-Diaminodiphenylmethan). Der Umsatz des 4,4′-Diaminodiphenylmethans beträgt 69%.

Beispiel 41

15 Teile eines handelsüblichen Gemisches aus 2,2′-, 2,4′- und 4,4′-Diaminodiphenylmethan und Polyphenyl-polymethylen-polyaminen werden mit 40 Teilen Carbamidsäureäthylester und 0,5 Teilen Kobaltacetat in 70 Teilen Äthanol 8 Stunden lang auf 190–195 °C erhitzt, wobei über ein Druckventil im Reaktionsgefäss ein Druck von 18–20 bar eingestellt wird. Das während der Reaktion gebildete Ammoniak wird unter Verwendung von 25 l Stickstoff je l Reaktionsgemisch und Stunde als Schleppmittel kontinuierlich abdestilliert. Man lässt erkalten und destilliert überschüssiges Äthanol und überschüssigen Carbamidsäureäthylester unter vermindertem Druck bei 10 mbar ab. Der Rückstand wird mit Wasser gewaschen, getrocknet und mit Cyclohexan versetzt und gerührt, wobei man einen pulverförmigen Niederschlag erhält, der abgetrennt und durch Hochdruck-Flüssigchromatographie analysiert wird. Es zeigt sich, dass ein Gemisch aus 2,4′-, 2,2′- und 4,4′-Di-(äthoxicarbonylamino)-diphenylmethan und Polyphenyl-polymethylen-polyäthylurethanen entstanden ist, das die gleichen Bestandteile enthält wie ein aus einer Mischung von 2,4′-, 2,2′- und 4,4′-Diisocyanatodiphenylmethan und Polyphenyl-polymethylen-polyisocyanaten mit Äthanol erhaltenes Vergleichsprodukt.

Beispiel 42

22 Teile 3-Aminophenol werden mit 100 Teilen Carbamidsäureäthylester, 12 Teilen Harnstoff, 1 Teil Zink-(II)-acetat und 45 Teilen Äthanol 8 Stunden lang auf 180–185 °C erhitzt, wobei über ein Druckventil im Reaktionsgefäss ein Druck von 7–8 bar eingestellt wird. Das während der Reaktion gebildete Ammoniak wird unter Verwendung von 20 l Stickstoff je l Reaktionsgemisch und Stunde als Schleppmittel kontinuierlich abdestilliert. Nach beendeter Reaktion analysiert man die Reaktionsmischung gaschromatographisch nach der Methode des «Internen Standard». Es zeigt sich, dass 29 Teile 3-Äthoxicarbonylaminophenol (90,2% der Theorie, bezogen auf umgesetztes 3-Aminophenol) entstanden sind. Der Umsatz des 3-Aminophenols beträgt 88%.

Beispiel 43

61 Teile 2,4-Diaminotoluol werden mit 432 Teilen Carbamidsäureoctylester und 1,5 Teilen Natriummethanolat in 1950 Teilen Octanol zum Sieden (195 °C) erhitzt. Nach 23 Stunden lässt man abkühlen und destilliert überschüssiges Octanol und überschüssigen Carbamidsäureoctylester bis zu einer Sumpftemperatur von 180 °C ab. Der Rückstand wird mit Hilfe der Hochdruck-Flüssigchromatographie nach der Methode des «Externen Standards» analysiert. Es zeigt sich, dass sich 57% des 2,4-Diaminotoluols umgesetzt haben, wobei 101 Teile (81,7% der Theorie) 2,4-Di-

(octoxicarbonylamino)toluol $C_{25}H_{42}O_4N_2$ (Molekulargewicht 434) und 10,9 Teile (13,8% der Theorie) eines Gemisches aus 2-Amino-4-(octoxicarbonylamino)toluol und 4-Amino-2-(octoxycarbonylamino)toluol gebildet haben.

Beispiel 44

40 Teile 2,4-Diaminotoluol werden mit 240 Teilen Carbamidsäurehexylester, 1,5 Teilen Kobaltacetat und 170 Teilen Hexanol 15 Stunden lang auf 155–175 °C erhitzt, wobei das entstehende Ammoniak kontinuierlich abdestilliert wird. Nach beendeter Reaktion untersucht man die Reaktionslösung durch Hochdruck-Flüssigchromatographie nach der Methode des «Externen Standards». Es zeigt sich, dass das 2,4-Diaminotoluol vollständig umgesetzt ist, wobei 119 Teile 2,4-Di-(hexoxicarbonylamino)-toluol (96% der Theorie, bezogen auf umgesetztes 2,4-Diaminotoluol) entstanden sind.

Beispiel 45

100 Teile eines handelsüblichen Roh-MDA-Gemisches, das zu 46% aus Diamino-diphenylmethan und zu 54% aus Polyphenyl-polymethylenpolyaminen besteht, werden mit 30,3 Teilen Harnstoff, 300 Teilen Carbamidsäurehexylester, 1,5 Teilen Kobaltacetat und 260 Teilen Hexanol 25 Stunden lang auf 155–175 °C erhitzt, wobei das entstehende Ammoniak kontinuierlich abdestilliert wird. Nach beendeter Reaktion untersucht man die Reaktionslösung durch Hochdruck-Flüssigchromatographie, wobei sich zeigt, dass ein Gemisch aus Di-(hexoxicarbonylamino)-diphenylmethanen und Poly-(hexoxicarbonylamino)-polyphenyl-polymethanen entstanden ist, welches identisch ist mit einem aus einer Mischung aus Diphenylmethan-diisocyanaten und Polyphenylpolymethylen-polyisocyanaten und Hexanol hergestellten Vergleichsprodukt.

Beispiel 46

50 Teile 4,4′-Diaminodiphenylmethan werden mit 360 Teilen Carbamidsäurehexylester, 1 Teil Kobaltacetat und 260 Teilen Hexanol auf 155–175 °C erhitzt. Man rührt 27 Stunden bei 155–175 °C. Nach dem Erkalten filtriert man vom abgesetzten Katalysator ab und analysiert die Reaktionsmischung durch Hochdruck-Flüssigchromatographie nach der Methode des «Externen Standards». Es zeigt sich, dass das 4,4′-Diaminodiphenylmethan vollständig umgesetzt ist, wobei 108 Teile 4,4′-Di-(hexoxicarbonylamino)-diphenylmethan (94,2% der Theorie, bezogen auf umgesetztes 4,4′-Diaminodiphenylmethan) entstanden sind. Die Reaktionslösung enthält ausserdem noch etwas 4-Amino-4′-(hexoxicarbonylamino)-diphenylmethan.

**Patentansprüche**

1. Verfahren zur Herstellung von Aryl-mono-, -di- und/oder -polyurethanen, dadurch gekennzeichnet, dass man primäre aromatische Mono-, Di- und/oder Polyamine mit O-Alkylcarbamidsäureestern in Gegenwart mindestens einer Verbindung, die ein oder mehrere Kationen von Metallen aus den Gruppen IA, IB, IIA, IIB, IIIA, IIIB, IVA, IVB, VA, VB, VIB, VIIB, VIIIB des Periodensystems besitzt, als Katalysator bei erhöhten Temperaturen umsetzt und das entstehende Ammoniak gegebenenfalls abtrennt.

2. Verfahren nach Anspruch 1, dadurch gekennzeichnet, dass man neben O-Alkylcarbamidsäureestern zusätzlich Alkohol mitverwendet.

3. Verfahren nach Ansprüchen 1 und 2, dadurch gekennzeichnet, dass man die Ausgangskomponenten in solchen Mengen umsetzt, dass pro $NH_2$-Gruppe der aromatischen Mono-, Di- und Polyamine 0,5 bis 20 Mole O-Alkylcarbamidsäureester und 0 bis 100 Mole Alkohol eingesetzt werden.

4. Verfahren nach Anspruch 1, dadurch gekennzeichnet, dass man neben O-Alkylcarbamidsäureestern zusätzlich Harnstoff und Alkohol mitverwendet.

5. Verfahren nach Anspruch 4, dadurch gekennzeichnet, dass man neben O-Alkylcarbamidsäureester maximal 1,5 Mole Harnstoff pro $NH_2$-Gruppe der Mono-, Di- und Polyamine einsetzt und das Molverhältnis von Harnstoff zu Alkohol gleich oder kleiner als 1 ist.

6. Verfahren nach Anspruch 1, dadurch gekennzeichnet, dass man als primäre aromatische Mono-, Di- und/oder Polyamine verwendet: Anilin, 3-Hydroxyanilin, 3,5-Dichloranilin, 2,4- und 2,6-Diamino-toluol und die entsprechenden Isomerengemische, 1,5-Diaminonaphthalin, 3,3′-Ditoluylen-4,4′-diamin, 2,2′-, 2,4′- und 4,4′-Diamino-diphenylmethan und die entsprechenden Isomerengemische und Mischungen aus Diamino-diphenylmethanen und Polyphenyl-polymethylen-polyaminen.

7. Verfahren nach Anspruch 1, dadurch gekennzeichnet, dass man als O-Alkylcarbamidsäureester solche aus Carbamidsäure und aliphatischen und cycloaliphatischen Monoalkoholen mit 1 bis 10 Kohlenstoffatomen im Alkoholrest verwendet.

8. Verfahren nach Anspruch 1, dadurch gekennzeichnet, dass man als Alkohole die den O-Alkylcarbamidsäureestern entsprechenden Alkohole verwendet.

9. Verfahren nach Anspruch 1, dadurch gekennzeichnet, dass man als Katalysatoren Verbindungen mit Kationen der Metalle Lithium, Calcium, Aluminium, Zinn, Wismut, Antimon, Kupfer, Zink, Titan, Vanadium, Chrom, Molybdän, Mangan, Eisen und Kobalt verwendet.

10. Verfahren nach Anspruch 1, dadurch gekennzeichnet, dass man die Umsetzung bei Temperaturen über 120 °C durchführt.

11. Verfahren nach Anspruch 1, dadurch gekennzeichnet, dass man gleichzeitig das entstehende Ammoniak abtrennt.

**Claims**

1. A process for the production of an aryl mono-, di- and/or polyurethane, wherein a primary aromatic mono-, di- and/or polyamine is reacted at elevated temperature with an O-alkylcarbamate in

the presence of at least one compound containing one or more cations of metals of groups IA, IB, IIA, IIB, IIIA, IIIB, IVA, IVB, VA, VB, VIB, VIIB and VIIIB of the periodic system, as catalyst, and the ammonia which forms may, if desired, be separated off.

2. A process as claimed in claim 1, wherein an alcohol is used in addition to the O-alkylcarbamate.

3. A process as claimed in claims 1 and 2, wherein the starting materials are reacted in such quantities that 0.5 to 20 moles of O-alkyl-carbamate and 0 to 100 moles of alcohol are employed per $NH_2$ group of the aromatic mono-, di- and polyamine.

4. A process as claimed in claim 1, wherein urea and an alcohol are used in addition to the O-alkylcarbamate.

5. A process as claimed in claim 4, wherein a maximum of 1.5 moles of urea per $NH_2$ group of the mono-, di- and polyamine are used in addition to the O-alkylcarbamate, and the molar ratio of urea to alcohol is equal to or less than 1.

6. A process as claimed in claim 1, wherein aniline, 3-hydroxyaniline, 3,5-dichloraniline, 2,4- and 2,6-diaminotoluene, the corresponding isomer mixtures thereof, 1,5-diaminonaphthalene, 3,3'-ditoluylene-4,4'-diamine, 2,2'-, 2,4'- and 4,4'-diaminodiphenylmethane and the corresponding isomer mixtures thereof, mixtures of diaminodiphenylmethanes and polyphenylpolymethylene polyamines are used as primary aromatic mono-, di- and/or polyamines

7. A process as claimed in claim 1, wherein the O-alkylcarbamates used are those of carbamic acids and aliphatic and cycloaliphatic monoalcohols having 1 to 10 carbon atoms in the alcohol radical.

8. A process as claimed in claim 1, wherein alcohols which correspond to the O-alkylcarbamate are used as alcohols.

9. A process as claimed in claim 1, wherein compounds containing cations of the metals lithium, calcium, aluminium, tin, bismuth, antimony, copper, zinc, titanium, vanadium, chromium, molybdenum, manganese, iron, and cobalt are used as catalysts.

10. A process as claimed in claim 1, wherein the reaction is carried out at temperatures above 120 °C.

11. A process as claimed in claim 1, wherein the ammonia which forms is separated off simultaneously.

**Revendications**

1. Procédé de préparation d'aryl-mono-, -di-et(ou) poly-uréthanes, caractérisé en ce que l'on fait réagir des mono-, di- et(ou) poly-amines aromatiques primaires et des esters d'acide O-alkyl-carbamique à des températures accrues en présence d'un composé comprenant un ou plusieurs cations de métaux des groupes IA, IB, IIA, IIB, IIIA, IIIB, IVA, IVB, VA, VB, VIB, VIIB et VIIIB du Système périodique des éléments comme catalyseur avec séparation éventuelle de l'ammoniac qui se forme.

2. Procédé suivant la revendication 1, caractérisé en ce qu'en plus des esters d'acide O-alkyl-carbamique, on emploie de l'alcool.

3. Procédé suivant les revendications 1 et 2, caractérisé en ce que l'on met en œuvre les composés de départ en des proportions telles qu'il y ait, par groupe $-NH_2$ des mono-, di- et poly-amines aromatiques, 0,5 à 20 moles d'esters d'acide O-alkyl-carbamique et 0 à 100 moles d'alcool.

4. Procédé suivant la revendication 1, caractérisé en ce que, à côté des esters d'acide O-alkyl-carbamique, on emploie en outre de l'urée et de l'alcool.

5. Procédé suivant la revendication 4, caractérisé en ce qu'à côté de l'ester d'acide O-alkyl-carbamique, on emploie au maximum 1,5 mole d'urée par groupe $-NH_2$ des mono-, di- et polyamines, le rapport molaire urée-alcool étant égal ou inférieur à 1.

6. Procédé suivant la revendication 1, caractérisé en ce que l'on emploie comme mono-, di-et(ou) polyamines aromatiques primaires l'aniline, la 3-hydroxy-aniline, la 3,5-dichlor-aniline, le 2,4- et le 2,6-diamino-toluène et les mélanges d'isomères correspondants, le 1,5-diamino-naphtalène, la 3,3'-ditoluylène-4,4'-diamine, le 2,2'-, 2,4'- et 4,4'-diamino-diphényl-méthane et les mélanges d'isomères correspondants et des mélanges de diamino-diphényl-méthanes et de polyphényl-polyméthylène-polyamines.

7. Procédé suivant la revendication 1, caractérisé en ce que l'on emploie comme esters d'acide O-alkyl-carbamique des esters de l'acide carbamique et de monols aliphatiques ou cycloaliphatiques avec un reste d'alcool en $C_1$ à $C_{10}$.

8. Procédé suivant la revendication 1, caractérisé en ce que l'on emploie comme alcools ceux qui correspondent aux esters d'acide O-alkyl-carbamique.

9. Procédé suivant la revendication 1, caractérisé en ce que l'on emploie comme catalyseurs des composés comportant des cations des métaux lithium, calcium, aluminium, étain, bismuth, antimoine, cuivre, zinc, titane, vanadium, chrome, molybdène, manganèse, fer et cobalt.

10. Procédé suivant la revendication 1, caractérisé en ce que la réaction est effectuée à des températures supérieures à 120 °C.

11. Procédé suivant la revendication 1, caractérisé en ce que l'ammoniac formé est éliminé simultanément.